# EUROPEAN PATENT SPECIFICATION

(11) **EP 0 392 459 B1**
(45) Date of publication and mention of the grant of the patent: **09.08.1995**
(21) Application number: 90106847.8
(22) Date of filing: 10.04.1990
(51) Int. Cl.: C12Q 1/48

(54) **Method for the measurement of reverse transcriptase by nonradioactive substance**
Verfahren zur Messung von Reverse-Transcriptase mit nichtradioaktiven Substanzen
Procédé pour mesurer la transcriptase inverse utilisant des substances non radio-actives

(30) Priority: 14.04.1989 JP 92948/89
(43) Date of publication of application: 17.10.1990
(73) Proprietor: ASAHI KASEI KOGYO KABUSHIKI KAISHA, Osaka (JP)
(72) Inventor: Mizoguchi, Junzo, Tagata-gun, Shizuoka-ken (JP); Nakai, Masuyo, Amagasaki-shi, Hyogo-ken (JP); Sano, Kouichi, Takatsuki-shi, Osaka-fu (JP)
(74) Representative: Wächtershäuser, Günter, Prof. Dr.

(56) References cited:
- EP-A- 0 186 526
- EP-A- 0 322 922
- FR-A- 2 611 274
- US-A- 4 879 214
- FEMS MICROBIOLOY LETTERS, vol. 48, nos. 1-2, December 1987, Elsevier; D.G. PITCHER et al., pp. 283-287/
- VIROLOGY, vol. 147, no. 2, December 1985, Academic Press, Inc.; A.D. HOFFMAN et al., pp. 326-335/
- PROCEEDINGS OF THE NATL. ACADEMY OF SCIENCES USA, vol. 78, no. 11, November 1981; P.R. LANGER et al., pp. 6633-6637/

## Description

### BACKGROUND OF THE INVENTION

### 1) Field of the Invention:

The present invention relates to a method for the determination of the presence of a reverse transcriptase in a sample liquid and also to a kit for the quantitation of the reverse transcriptase, said kit being useful in the practice of this method.

### 2) Description of the Related Art:

For the measurement of the activity of a reverse transcriptase, there has been reported a method in which a native RNA or adenine ribopolynucleotide (may hereinafter be abbreviated as "poly A") and an oligodeoxythyminenucleotide (may hereinafter be abbreviated as "oligo dT") and as a substrate, a tritium labeled deoxythymidine 5'-triphosphate (may hereinafter be abbreviated as "[³H]dTTP") are used, the oligo dT is allowed to elongate using the activity of the reverse transcriptase in a reaction mixture, the reaction mixture is filtered by a filter, and the radioactivity of the filter is then measured [David Baltimore, Nature, 226, 1209-1211 (1970); Howard M. Temin & Satoshi Mizutani, Nature, 226, 1211-1213 (1970)].

There has also been reported a method for the prognosis of AIDS, a symptom caused-by a sort of retrovirus, by making use of the above-mentioned measurement method of the activity of a reverse transcriptase [Japanese Patent Application (Publication) (Kokai) No. 252253/1988].

Many of the procedures which have heretofore been used commonly in biopharmaceutical research and recombinant DNA technology greatly rely upon the use of polynucleotides generally radiolabelled by isotopic hydrogen (³H), phosphorus (³²P), carbon (¹⁴C) or iodine (¹²⁶I). Such radioactive elements are employed as useful indicator probes because they can detect, monitor, localize or isolate nucleic acids or other scientifically or clinically interesting molecules even when such nucleic acids or molecules are present in a trace amount. Stringent limitations are however imposed on the use of radioactive substances, resulting in serious drawbacks. As a first problem, for those handling a radioactive substance, there is a potential danger that they can be exposed to high-level radiation. Careful and fail-free safety measures must be practiced during the production, use and post treatment of radioactive isotopes. A second problem resides in that a radioactive nucleotide is expensive and its use results in a higher cost in general applications. As a third problem, a radioactive substance of high specific radioactivity must be used to obtain a necessary level of sensitivity. The radioactive substance of high specific radioactivity however has a short half-life correspondingly, so that its shelf life is limited. This imposes limitations on its use.

It has hence been studied to use a nonradioactive labeling substance in place of such a radioactive substance. It has however been reported, for example, that when a native mRNA as a template, oligo dT as a primer and a biotinylated deoxyuridine 5'-triphosphate (may hereinafter be abbreviated as "biotin-dUTP") as a substrate were reacted using a reverse transcriptase derived from avian myeloma, biotin-dUTP was not used at all as a substrate, no elongation of oligo dT was observed, and biotin-dUTP cannot therefore be used for the measurement of the activity of the reverse transcriptase [Pennina R. Langer, Alex A. Waldrop & David C. Ward, Proc. Natl. Acad. Sci. USA, 78, 6633-6637 (1981)]. Biotinylated nucleotides have therefore been considered to be unapplicable for the measurement of the activities of reverse transcriptases.

### SUMMARY OF THE INVENTION

Under the aforementioned circumstances, there is an outstanding demand for the establishment of a system which enables the quantitation of the activity of a reverse transcriptase without using a radioactive isotope.

The present inventors have conducted an extensive investigation with a view toward obtaining a measurement system for the activity of a reverse transcriptase without using any radioactive isotope. As a result, it has been found that the use of a specific RNA template and a particular primer makes it possible to use biotin-dUTP as a substrate in the presence of a reverse transcriptase and the activity of the reverse transcriptase can thus be easily measured, leading to the completion of the present invention.

In one aspect of the present invention, there is thus provided a method for the determination of the presence of a reverse transcriptase in a sample liquid, which comprises reacting at least a hybridization product of an artificially-prepared adenine ribopolynucleotide RNA template and an oligodeoxythyminenucleotide complementary to the RNA template with biotinylated deoxyuridine triphosphate in the presence of the sample liquid; and after separation of the reaction product and the unreacted biotinylated deoxyuridine triphosphate from each other if necessary, quantitating the amount of the reacted or unreacted biotinylated deoxyuridine triphosphate.

In a further aspect of the present invention, there is also provided a kit for the quantitation of the amount of a reverse transcriptase, which kit is useful in the practice of the above methods. The kit comprises:
(i) an artificially-prepared adenine ribopolynucleotide RNA template;
(ii) an oligodeoxythyminenucleotide complementary to the RNA template;
(iii) biotinylated deoxyuridine triphosphate; and
(iv) a reagent for the quantitation of the amount of biotin.

The present invention has made it possible to detect the activity of a reverse transcriptase, for example, in the serum of an AIDS virus carrier without using any radioactive labeling substance. It has also become possible to quantitate the amount of the reverse transcriptase without the need for any radioactive labeling substance. It is therefore feasible to easily monitor the danger of development of AIDS symptoms by AIDS virus carriers.

Good sensitivity is available when the molar ratio of the poly A to the oligo dT is set at 1 to 1.5-2,000,000. It is more preferable to use the oligo dT at a molar ratio of 15-20,000 relative to the poly A. It is possible to conduct measurements with highest sensitivity when the molar ratio of the poly A to the oligo dT is set at 1 to 150-2,000.

The additional use of dTTP is extremely preferred. When dTTP is added at the above preferred molar ratio of the poly A to the oligo dT, an extremely sensitive measurement is feasible.

### DESCRIPTION OF THE INVENTION AND PREFERRED EMBODIMENTS

In the present invention, the RNA employed as a template is artificially-prepared adenine ribopolynucleotide. Such an RNA may hereinafter be abbreviated as "poly A".

Poly A can be artificially prepared, for example, by synthesizing the same using a gene manipulation technique or chemical synthesis technique. Poly As made of 100-500 bases can be mentioned as particularly preferred examples. Poly A of about 250 bases long on average can be mentioned as an example that can usually be employed advantageously.

Oligodeoxythyminenucleotide (may hereinafter be abbreviated as "oligo dT") acts as a primer. Deoxythymidine oligomers made of 5-25 bases can be mentioned as preferred examples. An oligo dT of about 15 bases long on average can be mentioned as an example that can be usually employed advantageously.

As the biotinylated deoxynucleotide triphosphate (may hereinafter be abbreviated as "biotin-dUTP" or a "labeling substrate") employed as a substrate, may be mentioned by way of example a compound in which a biotin group and a uracyldeoxynucleoside group are coupled together via a suitable spacer such as aminoaryl group. Exemplary specific compounds include compounds in which a biotinyl group is coupled with dUTP via a suitable spacer, such as 5-[N-(N-biotinyl-ε-aminocaproyl)-3-aminoaryl]deoxyuridine triphosphates (biotin-11-dUTPs) and 5-[N-[N-(biotinyl-ε-aminocaprolyl)-γ-aminobutyryl]-3-aminoaryl]-deoxyuridine triphosphates (biotin-16-dUTP). Biotin-11-dUTPs and biotin-16-dUTPs are compounds available on the market, and other compounds can also be easily prepared in accordance with the processes disclosed in known publications such as Japanese Patent Application Laid-Open (Kokai) No. 209297/1982.

As reagents useful for the quantitation of the amount of biotin and compositions thereof, it is possible to use those generally employed for the quantitation of the amount of biotin. Illustrative examples include fluorescein isothiocyanate (FITC) fluorescence detecting compositions, peroxidase coloring and detecting compositions, acidic phosphatase coloring and detecting compositions, alkaline phosphatase coloring and detecting compositions, and β-galactosidase coloring and detecting compositions.

Exemplary FITC fluorescence detecting compositions include compositions comprising rabbit anti-biotin antibody IgG, FITC-conjugated goat anti-rabbit antibody IgG and Evans blue as well as compositions comprising FITC-conjugated avidin and Evans blue. Illustrative peroxidase coloring and detecting compositions include compositions comprising a streptavidin-biotinylated horseradish peroxidase complex, a suitable buffer such as phosphate buffer, and cobalt chloride.

Further, exemplary acidic phosphatase coloring and detecting compositions include a streptavidin-biotinylated acidic phosphatase complex, a buffer such as phosphate buffer, naphthol AS-MX phosphate and fast violet B salt. while illustrative alkaline phosphatase coloring and detecting compositions include compositions comprising a streptavidin-biotinylated alkaline phosphatase complex, a suitable buffer such as tris-HCl buffer, 5-bromo-4-chloro-3-indoylphosphate, and nitroblue tetrazolium as well as compositions comprising a streptavidin alkaline phosphatase, a suitable buffer such as tris-HCl, 5-bromo-4-chloro-3-indolylphosphate, and nitroblue tetrazolium. In addition, exemplary β-galactosidase coloring and detecting compositions include compositions comprising a streptavidin-β-galactosidase complex, a suitable buffer such as phosphate buffer and 2-nitrophenyl-β-galactoside. As these compositions, it is possible to use those already available on the market or reagent compositions for the quantitation of biotin, said reagent compositions having been prepared in various ways with reference to known publications.

To practice the method of the present invention for the determination of the presence of a reverse transcriptase in a sample liquid, it is firstly necessary to hybridize the poly A with the oligo dT to obtain a hybridization product. This hybridization can be conducted by reacting the poly A and the oligo dT in a buffer such as tris-HCl buffer or phosphate buffer, said buffer being controlled at 4-37°C and pH 6.5-8°C, by a method known *per se* in the art.

Regarding the quantitative relation between the poly A (average strand length: 250 bases) and the oligo dT (average strand length: 15 bases), it is preferred to use the poly A and the oligo dT at a molar ratio of 1 to 1.5-2,000,000. Use of the poly A and the oligo dT at a molar ratio of 1 to 15-20,000 is more preferred as this molar ratio will produce a color with a high intensity and hence permits easy measurement. In particular, it is most preferred to use the poly A and the oligo dT at a molar ratio of 1 to 150-2,000 because an extremely easy measurement is feasible.

This hybridization step can be conducted as an independent step or concurrently with the next step in which the primer is subjected to an elongation reaction by the reverse transcriptase.

Next, using at least the biotin-dUTP as a labeling substrate, the biotin-dUTP is incubated around 37°C for 0.5-24 hours along with a buffer, such as tris-HCl buffer or phosphate buffer having been adjusted to pH 6.5-8, which contains the sample liquid and the hybridization product of the poly A and the oligo dT, whereby the biotin-dUTP is coupled to the primer site of the poly A-oligo dT under the action of the reverse transcriptase present in the system.

Any solution can be used as the sample liquid as long as it is intended to determine the presence or absence of a reverse transcriptase in the solution or to measure the enzymatic activity of the reverse transcriptase in the solution. Cultured media of retrovirus can be mentioned by way of example.

Further, if necessary, the biotin-dUTP coupled with the poly A-oligo dT (may hereinafter be referred to as the "reacted biotin-dUTP") is separated from the unreacted biotin-dUTP, and the amount of the reacted or unreacted biotin-dUTP is quantitated.

No particular limitation is imposed on the method for the separation of the reacted and unreacted biotin-dUTP. They can be separated from each other, for example, by passing the reaction mixture through a microporous filter which does not permit the passage of the reacted and elongated biotin-dUTP coupled with the poly A-oligo dT through the reaction step but allows the passage of the reaction medium and the unreacted substrate.

Illustrative of the microporous filer include glass microfibra filters ("WHATMAN GF/A"™, trade name; product of Whatman Inc.) and nylon membranes ("BIODYNE"™, trade mark; product of Japan Pall Co., Ltd.).

They can also be separated from each other, for example, by acid-alkaline electrophoresis which uses a gel membrane such as an agarose gel or acrylamide gel. Namely, the reaction mixture is subjected to electrophoresis through a gel membrane under acidic conditions, whereby the hybridized product of the primer elongated through the reaction step with the poly A, the large RNA employed as the template, is retained on the surface of the membrane and/or within the membrane while the unreacted substrate, reaction medium and the like are allowed to pass through the membrane. The membrane is then subjected to electrophoresis under alkaline conditions, so that only the primer which has been subjected to the elongation step is caused to pass through the membrane and is therefore separated.

The amount of the biotinylated deoxyuridine employed as a substrate, namely, the amount of the reacted biotin-dUTP can be determined by quantitating, with a biotin-quantitating reagent, the amount of biotin in the biotinylated deoxyuridine coupled with the primer through the elongation step. When a composition comprising a streptavidin alkaline phosphatase, tris-HCl buffer, 5-bromo-4-chloro-3-indolylphosphate (BCIP) and nitroblue tetrazolium (NBT) is used as an exemplary reagent for the quantitation of the content of biotin, the quantitation of the amount of the reacted biotin-dUTP can be practiced in the following manner. A microporous filter is immersed for 2-30 minutes in a solution which has been obtained by adding a streptavidin alkaline phosphatase to a concentration of 0.1-10 »g/mℓ in tris-HCl buffer containing 0.15 M of NaCl and controlled at pH 6.8-7.5. The thus-immersed microporous filter is washed with a sufficient amount of the above buffer at least 3 times and is then immersed for 10 minutes or longer in tris-HCl buffer which contains 0.1 M of NaCl and 50 mM of MgCl₂ and has been controlled to pH 8.5-10. The filter is thereafter immersed for 0.1-2 hours in a solution which has been obtained by adding NBT and BCIP to concentrations of 0.1-1 mg/mℓ and 0.1-1 mg/mℓ, respectively to the aforementioned buffer, whereby the filter is caused to develop a color. No particular limitation is imposed on the method for the measurement of the intensity of the color thus produced, as long as the absorbance can be measured. For example, when a microporous filter is employed, its color intensity can be measured by a densitometer, using usually a wavelength of 400-600 nm, preferably a wavelength of 500-550 nm.

Since the coupled amount of the biotin-dUTP thus measured reflects the amount of the reverse transcriptase in the system, namely, the amount of the reverse transcriptase in the sample liquid, the amount of the reverse transcriptase or the like can be determined therefrom.

To increase the detection sensitivity, it is also possible to add deoxythymidine triphosphate (may hereinafter be abbreviated "dTTP") as a substrate in addition to the labeling substrate. For this purpose, the dTTP can be used normally at a molar ratio of 2-20 times relative to the amount of the biotin-dUTP used as the labeling substrate.

The quantitative determination of the amount of the reverse transcriptase by the method of the present invention has good sensitivity so that the reverse transcriptase as little as 0.001-1 unit or so can be usually quantitated.

Upon practice of a method for the determination of infection to a retrovirus on the basis of a body fluid, it is firstly necessary to obtain the hybridization product of the poly A and the oligo dT like the above-described method for the determination of the presence of the reverse transcriptase. This step can be carried out in a similar manner to the step described above.

Next, using at least the biotin-dUTP as a substrate, the biotin-dUTP is reacted with the hybridization product of the poly A and the oligo dT in the presence of a sample liquid and a reverse transcriptase. Upon practice of this step, it is preferred from the standpoint of the accuracy of measurement that the sample liquid and the reverse transcriptase are sufficiently reacted in advance. Described more specifically, a solution containing a reverse transcriptase whose enzymatic activity has already been measured is prepared usually in an amount of 0.001-1 unit, preferably in an amount of 0.001-0.01 unit. The reverse-transcriptase-containing solution and the sample liquid are preincubated at about 4°C usually for 0.1-2 hours, preferably for 10-60 minutes to have them reacted sufficiently. It is then necessary to incubate at about 37°C for 0.5-6 hours or so the reaction mixture of reverse transcriptase-sample liquid along with the above-described hybridization product and a buffer, such as Tris-HCl buffer having been adjusted to pH 6.5-8, which contains usually 0.5-100 »M, preferably 1-5 »M of biotin-dUTP as a substrate. Incubation for 6-18 hours or so is more preferred because measurement errors can be minimized.

Exemplary usable reverse transcriptases include those obtained by subjecting viral particles of retrovirus - such as human immunodeficiency virus type 1 (HIV-1), human immunodeficiency virus type 2 (HIV-2), simian immunodeficiency virus (SIV), mouse mammary tumor virus, human T-cell leukemia virus type I (HTLV-1), Rous avian sarcoma virus, Rous associated virus type 2 (PAV-2)and HBV (hepatitis B virus) - to solubilize with a non-ionic surfactant (e.g., "Triton™ X-100", trade name; and "NP-40"™, trade name).

On the other hand, the sample liquid may be any desired body fluid which is either contained or produced in the human body having an antibody against the reverse transcriptase. Serum is preferred.

The serum is normally employed in a form precipitated with a 30-50% saturated ammonium sulfate solution or in a form passed through a cation-exchange resin such as DEAE or QAE-Sephadex®.

The separation of the reacted and unreacted biotin-dUTP and the quantitation of their amounts can be conducted following the procedure of the above-described method for the determination of the presence of the reverse transcriptase.

The amount of the reacted or unreacted biotin-dUTP in the measurement system can be determined as described above. It is to be noted that the reverse transcriptase in the system is deactivated by the antibody introduced from the sample liquid and as a result, the amount of the reacted biotin-dUTP is decreased. Accordingly, the reverse transcriptase neutralizing and/or inhibiting antibody titer of the body fluid can be determined using as an index a positive control body fluid and a negative control body fluid. This antibody titer can be used as an index for the determination of infection or non-infection to the retrovirus on the basis of the body fluid.

It is also preferred to use the oligo dT (average strand length: 15 bases) at a molar ratio of 1.5-2,000,000 relative to the poly A (average strand length: 250 bases). It is more preferred to use the oligo dT at a molar ratio of 15-20,000 relative to the poly A, because a color can be produced with high intensity and can hence be measured easily.

Especially, it is most preferred to use the oligo dT at a molar ratio of 150-2,000 relative to poly A since the measurement of the resulting color intensity is extremely easy.

To increase the detection sensitivity, it is also possible to add the dTTP as a substrate in addition to the labeling substrate. For this purpose, dTTP can be used normally at a molar ratio of 2-20 times relative to the amount of the biotin-dUTP used as the labeling substrate.

To advantageously practice a method for the determination of infection to a retrovirus, it is possible to use, for example, a kit of the present invention for the quantitation of the amount of a reverse transcriptase, which comprises:
(i) an artificially-prepared adenine ribopolynucleotide RNA template;
(ii) an oligodeoxythyminenucleotide complementary to the RNA template;
(iii) a biotinylated deoxyuridine triphosphate; and
(iv) a reagent for the quantitation of the amount of biotin.

As to the quantitative relation between the poly A and the oligo dT in the kit of the present invention, the molar ratio of the oligo dT to the poly A can be set preferably at 1.5-2,000,000 to 1, notably at 150-2,000 to 1. The kit therefore contains the poly A and the oligo dT in suitable amounts satisfying the above molar ratio. Described specifically, the exemplary preferred amounts are 0.1-10 »g/mℓ of the poly A and 10-1,000 »g/mℓ of the oligo dT.

On the other hand, the amount of the biotin-dUTP is normally 0.5-100 »M, with 1-5 »M being preferred.

It is also preferred to incorporate dTTP, usually, in an amount at a molar ratio 2-20 times the amount of the biotin-dUTP in the kit of the present invention, because the detection sensitivity can be increased.

In these kits, each reagent may be in the form of a solution. As an alternative, each reagent may be in a lyophilized form so that it can be dissolved with deionized water, physiological saline or one of various buffers upon use.

The present invention will hereinafter be described in further detail. It should however be borne in mind that the present invention is not necessarily limited to or by the following examples.

### Example 1

(1) DNA strand elongation reaction by reverse transcriptase at various mixing ratios of poly A to oligo dT:
To conduct DNA strand elongation reactions by a reverse transcriptase, were prepared 100 »ℓ reaction solutions which commonly contained a buffer [50 mM tris-HCl buffer (pH 7.8), 12 mM of dithiothreitol, 1.2 mM of reduced glutathione, 10 mM of MgCl₂, 160 mM of KCl, 1 mM of ethylene glycol tetraacetic acid, 0.2% of "Triton X-100"™ (trade name) and 2% of ethylene glycol], 10 »g/mℓ of oligo dT₁₂₋₁₈ (15 bases long on average; product of Pharmacia AB), and 4 »M of biotin-11-dUTP (product of ENZO Company) and also contained a poly A (250 bases long on average; product of Pharmacia AB) at concentrations of 10⁻⁴ »g/mℓ, 10⁻³ »g/mℓ, 10⁻² »g/mℓ, 10⁻¹ »g/mℓ, 10⁰ »g/mℓ, 10¹ »g/mℓ, 10² »g/mℓ, 10³ »g/mℓ and 0 »g/mℓ, respectively. 15 mU of a reverse transcriptase (product of Takara Shuzo Co., Ltd.; derived from Rous associated virus 2) were added to and mixed with each of the reaction solutions. The resultant mixture was incubated at 37°C for 18 hours.
(2) Adsorption of elongated DNA strands on nylon membranes:
Each of the reaction mixtures obtained as described in the above procedure (1) was added with a tenfold amount of a solution which contained 1N hydrochloric acid and 0.1 M of sodium pyrophosphate. The resultant mixture was stirred and then ice-cooled for 10 minutes. The solution thus obtained was subjected to suction filtration through a 100 cm² wide nylon membrane ("BIODYNE"™, trade mark; product of Japan Pall Co., Ltd.) mounted on a 96-aperture manifold (manufactured by Bethesda Research Laboratories), whereby elongated biotinylated DNA strands were adsorbed on the nylon membrane.
(3) Quantitation of biotinylated DNAs:
Nylon membranes which were obtained in the above procedure (2) and carried biotinylated DNA strands adsorbed thereon were each added with 3% BSA dissolved in Buffer 1 [a buffer consisting of 0.1 M tris-HCl buffer (pH 7.5) and 0.15 M NaCl], followed by incubation at 65°C for 1 hour. The nylon membrane with the biotinylated DNA strands adsorbed thereon was taken out of the buffer, and was then placed for 10 minutes in a solution which had been obtained by adding 1 »g/mℓ of a streptavidin alkaline phosphatase ("BLUE GENE"™, trade mark; product of Bethesda Research Laboratories). The solution was employed in an amount of 7 mℓ per 100 cm² of the nylon membrane. The nylon membrane was thereafter exposed for 15 minutes to 100 mℓ of a buffer. The nylon membrane was then caused to adsorb biotinylated DNA strands twice in a similar manner, followed by exposure to 100 mℓ of a buffer for 15 minutes.
The nylon membrane with the biotinylated DNA strands adsorbed thereon was taken out and washed for 10 minutes with 100 mℓ of Buffer 2 [a buffer consisting of 0.1 M tris-HCl buffer (pH 9.5), 0.1 M NaCl and 50 mM of MgCl₂].
The visualization of biotinylated DNA strand spot was conducted by placing the nylon membrane for 30 minutes in Buffer 3 which contained 0.33 mg/mℓ of nitroblue tetrazolium (NBT) and 0.17 mg/mℓ of 5-bromo-4-chloro-3-indolylphosphate (BCIP). Buffer 3 was used in an amount of 7.5 mℓ per 100 cm² of the nylon membrane. Color spots developed on the nylon membrane were measured at the wavelength of 550 nm by means of a densitometer ("CS-930"™, trade name; manufactured by Shimadzu Corporation).
The area intensities shown in Table 1 were obtained when 15 mU of the reverse transcriptase and 10 »g/mℓ of oligo dT₁₂₋₁₈ were used and reacted at 37°C for 18 hours by respectively using the reaction solution not added with the poly A and the reaction solutions containing the poly A at 10⁻⁴ »g/mℓ, 10⁻³ »g/mℓ, 10⁻² »g/mℓ, 10⁻¹ »g/mℓ, 10⁰ »g/mℓ, 10¹ »g/mℓ, 10² »g/mℓ and 10³ »g/mℓ.

**Table 1**

| Amount of poly A (»g/mℓ) | Area intensity (x 10³) |
|---|---|
| 0 | 0 |
| 10⁻⁴ | 5.7 |
| 10⁻³ | 11 |
| 10⁻² | 27 |
| 10⁻¹ | 38 |
| 10⁰ | 45 |
| 10¹ | 24 |
| 10² | 3.5 |
| 10³ | 0.8 |

As is apparent from the above table, the strongest color was produced when the poly A was used at the concentration of 1 »g/mℓ per 10 »g/mℓ of oligo dT₁₂₋₁₈, namely, when the poly A and oligo dT₁₂₋₁₈ were used at the weight ratio of 10:1, i.e., at the molar ratio of 167:1.
The area intensities shown in Table 2 were obtained when oligo dT₁₂₋₁₈ and the poly A were used at the concentrations of 10 »g/mℓ and 1 »g/mℓ, respectively and reactions were conducted at 37°C for 18 hours by respectively using the reaction solutions containing 1.5 mU, 15 mU and 150 mU of the reverse transcriptase.

**Table 2**

| Amount of enzyme (mU) | Area intensity (x 10³) |
|---|---|
| 1.5 | 2.5 |
| 15 | 42 |
| 150 | 370 |

When reactions were conducted at 37°C for 1, 2, 3 and 24 hours, respectively while using 15 mU of the enzyme, the area intensities after the respective reaction times were as shown in Table 2. The reaction was found to have time dependency.

**Table 3**

| Reaction time (hr) | Area intensity (x 10³) |
|---|---|
| 1 | 2.3 |
| 2 | 7.3 |
| 3 | 9.2 |
| 24 | 45 |

(4) Quantitation of antibody against reverse transcriptase:
Molt-4 cells (purchased from Dainippon Pharmaceutical Co., Ltd.) were infected to human immunodeficiency virus (HIV). The cells were cultured under the carbon dioxide gas concentration of 5% and 37°C in RMPI-1640 medium (product of Gibco) to which fetal calf serum had been added at the concentration of 5%. The supernant of the culture medium was centrifuged at 30,000 rpm for 1 hour, whereby a pellet of viral particles (10⁶ particles) was obtained. The pellet was suspended in a buffer which consisted of 0.5% of "Triton X-100"™, 0.8 mM of NaCl, 0.5 mM of phenylmethylsulfonyl fluoride, 20% of glycerin and 50 mM tris-HCl buffer (pH 7.8), so that an enzyme solution was prepared.
On the other hand, serum collected from an HIV infected patient was precipitated with a 40% saturated ammonium sulfate solution. The serum thus partially purified was used as a reverse transcriptase antibody, which contained IgG at the total concentration of 500 »g per mℓ. First, 25 »ℓ of the stock, 1/10 dilution and 1/100 dilution of the antibody solution and water were mixed respectively with 25 »ℓ portions of the enzyme solution described above, followed by incubation at 4°C for 30 minutes. An enzyme reaction solution whose concentration was twice the concentration of the reaction solution described in the procedure (1) was prepared. The thus incubated enzyme-antibody mixtures were added with 50 »ℓ portions of the enzyme reaction solution of the twofold concentration, respectively. After mixing the resultant mixtures, they were incubated further at 37°C for 18 hours. The activities of the reverse transcriptase remaining in the mixtures were separately measured by the measurement method described in the procedures (2)-(3). As a result, the area intensities shown in Table 4 were obtained. The reverse transcriptase antibody contained in the serum was therefore quantitated.

**Table 4**

| Dilution of antibody (times) | Area intensity (x 10³) |
|---|---|
| x 1 | 0.2 |
| x 10 | 80 |
| x 100 | 390 |
| (No antibody) | 400 |

(5) Increase of detection sensitivity for biotinylated DNA by the addition of dTTP:
50 »ℓ portions of the enzyme reaction solution of the twofold concentration containing 4 »M of biotin-11-dUTP described in the procedure (1) were furnished. They were separately added with dTTP to give the final concentrations of 8, 16, 40 and 80 »M of the latter. The resultant mixtures were each added with 1.5 mU of the reverse transcriptase, followed by the addition to water to the total volume of 100 »ℓ. After they were reacted at 37°C for 18 hours, the resulting mixtures were measured in accordance with the measuring methods described above in the procedures (2)-(4). As a result, the area intensities shown in Table 5 were obtained. From the result, the detection sensitivity for the biotinylated DNA was found to be the best when biotin-11-dUTP and dTTP were mixed at the molar concentration ratio of 1:4.

**Table 5**

| dTTP (»M) [bio-11-dUTP:dTTP] | Area intensity (x 10³) |
|---|---|
| 0 [1:0] | 2.5 |
| 8 [1:2] | 5 |
| 16 [1:4] | 7 |
| 40 [1:10] | 6 |
| 80 [1:20] | 5.5 |

(6) Further, 50 »ℓ portions of the biotin-11-dUTP-free enzyme reaction solution of the twofold concentration out of the compositions described above in the procedure (1) were furnished. They were individually added with biotin-11-dUTP and dTTP to give mixtures in which biotin-11-dUTP and dTTP were contained at the final concentrations 4 and 0 »M, 4 and 8 »M, 2 and 10 »M, 1 and 11 »M and 0.5 and 11.5 »M, respectively. Each of the resultant mixtures was added with 1.5 mU of the reverse transcriptase and further with water to the total volume of 100 »ℓ. They were measured by the measuring methods described above, whereby the area intensities shown in Table 6 were obtained. It is understood from the area intensities that the detection intensity for the biotinylated DNA is the best when biotin-11-dUTP and dTTP are used in combination at molar concentrations of 2 »M and 10 »M, respectively.

**Table 6**

| Biotin-11-dUTP (»M) | dTTP (»M) | Area intensity (x 10³) |
|---|---|---|
| 4 | 0 | 2.5 |
| 4 | 8 | 5 |
| 2 | 10 | 6.9 |
| 1 | 11 | 5.5 |
| 0.5 | 11.5 | 4.5 |

## Claims

1. A method for the determination of the presence of a reverse transcriptase in a sample liquid, which comprises reacting at least a hybridization product of an artificially-prepared adenine ribopolynucleotide RNA template and an oligodeoxythyminenucleotide complementary to the RNA template with a biotinylated deoxyuridine triphosphate in the presence of the sample liquid; and after separation of the reaction product and the unreacted biotinylated deoxyuridine triphosphate from each other if necessary, quantitating the amount of the reacted or unreacted biotinylated deoxyuridine triphosphate.

2. The method of claim 1, wherein the RNA template is made of 100-500 bases, the oligodeoxythyminenucleotide is made of 5-25 bases, and the hybridization product has been formed by hybridizing the RNA template and the oligodeoxythyminenucleotide at a molar ratio of 1 to 1.5-2,000,000.

3. The method of claim 1, wherein the RNA template is made of 100-500 bases, the oligodeoxythyminenucleotide is made of 5-25 bases, and the hybridization product has been formed by hybridizinc the RNA template and the oligodeoxythyminenucleotide at a molar ratio of 1 to 15-20,000.

4. The method of claim 1, wherein the RNA template is made of 100-500 bases, the oligodeoxythyminenucleotide is made of 5-25 bases, and the hybridization product has been formed by hybridizing the RNA template and the oligodeoxythyminenucleotide at a molar ratio of 1 to 150-2,000.

5. The method of claim 1, wherein deoxythymidine triphosphate is additionally contained upon conducting the reaction.

6. A kit for carrying out a method according to Claims 1 to 5 which comprises:
(i) an artificially-prepared adenine ribopoly-nucleotide RNA template;
(ii) an oligodeoxythyminenucleotide complementary to the RNA template;
(iii) a biotinylated deoxyuridine triphosphate; and
(iv) a reagent for the quantitation of the amount of biotin.

7. The kit of claims 6, wherein the RNA template is made of 100-500 bases, the oligodeoxythyminenucleotide is made of 5-25 bases, and the RNA template and oligodeoxythyminenucleotide are contained as a hybridization product formed by hybridizing the RNA template and the oligodeoxythyminenucleotide at a molar ratio of 1 to 1.5-2,000,000.

8. The kit of claim 6, wherein the RNA template is made of 100-500 bases, the oligodeoxythyminenucleotide is made of 5-25 bases, and the RNA template and oligodeoxythyminenucleotide are contained as a hybridization product formed by hybridizing the RNA template and the oligodeoxythyminenucleotide at a molar ratio of 1 to 15-20,000.

9. The kit of claim 6, wherein the RNA template is made of 100-500 bases, the oligodeoxythyminenucleotide is made of 5-25 bases, and the RNA template and oligodeoxythyminenucleotide are contained as a hybridization product formed by hybridizing the RNA template and the oligodeoxythyminenucleotide at a molar ratio of 1 to 150-2,000.

10. The kit of claim 6, further comprising:
(v) deoxythymidine triphosphate.

## Patentansprüche

1. Verfahren zum Bestimmen der Anwesenheit einer reversen Transcriptase in einer Probenflüssigkeit, umfassend das Umsetzen mindestens eines Hybridisierungsproduktes einer künstlich hergestellten Adenin-Ribopolynucleotid-RNA-Matrize und eines Oligodesoxythymin-Nucleotids, das komplementär zur RNA-Matrize ist, mit einem biotin-markierten Desoxyuridin-triphosphat in Gegenwart der Probenflüssigkeit und, nach der Trennung des Reaktionsproduktes und des nicht umgesetzten biotin-markierten Desoxyuridin-triphosphats voneinander, falls erforderlich, quantitatives Bestimmen der Menge des umgesetzten oder nicht umgesetzten biotin-markierten Desoxyuridin-triphosphats.

2. Verfahren nach Anspruch 1, worin die RNA-Matrize aus 100 bis 500 Basen, das Oligodesoxythymin-Nucleotid aus 5 bis 25 Basen besteht und das Hybridisierungsprodukt durch Hybridisieren der RNA-Matrize und des Oligodesoxythymin-Nucleotids in einem molaren Verhältnis von 1 bis 1,5-2.000.000 gebildet wurde.

3. Verfahren nach Anspruch 1, worin die RNA-Matrize aus 100 bis 500 Basen, das Oligodesoxythymin-Nucleotid aus 5 bis 25 Basen besteht, und das Hybridisierungsprodukt durch Hybridisieren der RNA-Matrize und des Oligodesoxythymin-Nucleotids in einem molaren Verhältnis von 1 bis 15-20.000 gebildet wurde.

4. Verfahren nach Anspruch 1, worin die RNA-Matrize aus 100 bis 500 Basen, das Oligodesoxythymin-Nucleotid aus 5 bis 25 Basen besteht, und das Hybridisierungsprodukt durch Hybridisieren der RNA-Matrize und des Oligodesoxythymin-Nucleotids in einem molaren Verhaltnis von 1 bis 150-2.000 gebildet wurde.

5. Verfahren nach Anspruch 1, worin Desoxythymidin-triphosphat zusätzlich beim Ausführen der Umsetzung vorhanden ist.

6. Ausrüstung zum Ausführen eines Verfahrens nach Anspruch 1 bis 5, umfassend:
(i) eine künstlich hergestellte Adenin-Ribopolynucleotid-RNA-Matrize;
(ii) ein Oligodesoxythymin-Nucleotid, das komplementär zur RNA-Matrize ist:
(iii) ein biotin-markiertes Desoxyuridin-triphosphat und
(iv) ein Reagenz zur quantitativen Bestimmung der Biotinmenge.

7. Ausrüstung nach Anspruch 6, worin die RNA-Matrize aus 100 bis 500 Basen, das Oligodesoxythymin-Nucleotid aus 5 bis 25 Basen besteht, und die RNA-Matrize und Oligodesoxythymin-Nucleotid als ein Hybridisierungsprodukt enthalten sind, gebildet durch Hybridisieren der RNA-Matrize und des Oligodesoxythymin-Nucleotids in einem molaren Verhaltnis von 1 bis 1,5-2.000.000.

8. Ausrüstung nach Anspruch 6, worin die RNA-Matrize aus 100 bis 500 Basen, das Oligodesoxythymin-Nucleotid aus 5 bis 25 Basen besteht, und die RNA-Matrize und Oligodesoxythymin-Nucleotid als ein Hybridisierungsprodukt enthalten sind, gebildet durch Hybridisieren der RNA-Matrize und des Oligodesoxythymin-Nucleotids in einem molaren Verhältnis von 1 bis 15-20.000.

9. Ausrüstung nach Anspruch 6, worin die RNA-Matrize aus 100 bis 500 Basen, das Oligodesoxythyrein-Nucleotid aus 5 bis 25 Basen besteht, und die RNA-Matrize und Oligodesoxythymin-Nucleotid als ein Hybridisierungsprodukt enthalten sind, gebildet durch Hybridisieren der RNA-Matrize und des Oligodesoxythymin-Nucleotids in einem molaren Verhältnis von 1 bis 150-2.000.

10. Ausrüstung nach Anspruch 6, weiter umfassend:
(v) Desoxythymidin-triphosphat.

## Revendications

1. Méthode de détermination de la présence d'une transcriptase inverse dans un liquide échantillon, qui comprend la mise en réaction d'au moins un produit d'hybridation d'une matrice d'ARN d'adénine ribopolynucléotide préparée artificiellement et d'un oligodésoxythyminenucléotide complémentaire de la matrice d'ARN avec un désoxyuridine triphosphate biotinylé en présence du liquide échantillon ; et après séparation du produit de réaction et du désoxyuridine triphosphate biotinylé n'ayant pas réagi l'un de l'autre si nécessaire, la quantification de la quantité de désoxyuridine triphosphate biotinylé ayant réagi et n'ayant pas réagi.

2. Méthode selon la revendication 1, dans laquelle la matrice d'ARN est constituée de 100 à 500 bases, l'oligo désoxythyminenucléotide est constitué de 5 à 25 bases, et le produit d'hybridation a été formé en hybridant la matrice d'ARN et l'oligodésoxythyminenucléotide à un rapport molaire de 1 à 1,5-2 000 000.

3. Méthode selon la revendication 1, dans laquelle la matrice d'ARN est constituée de 100 à 500 bases, l'oligodésoxythyminenucléotide est constitué de 5 à 25 bases, et le produit d'hybridation a été formé en hybridant la matrice d'ARN et l'oligodésoxythyminenucléotide à un rapport molaire de 1 à 15-20 000.

4. Méthode selon la revendication 1, dans laquelle la matrice d'ARN est constituée de 100 à 500 bases, l'oligodésoxythyminenucléotide est constitué de 5 à 25 bases, et le produit d'hybridation a été formé en hybridant la matrice d'ARN et l'oligodésoxythyminenucléotide à un rapport molaire de 1 à 150-2000.

5. Méthode selon la revendication 1, dans laquelle le désoxythymidine triphosphate est contenu en plus lors de la mise en oeuvre de la réaction.

6. Kit pour mettre en oeuvre une méthode selon les revendications 1 à 5 qui comprend :
(i) une matrice d'ARN d'adénine ribopolynucléotide préparée artificiellement,
(ii) un oligodésoxythyminenucléotide complémentaire de la matrice d'ARN ;
(iii) un désoxyuridine triphosphate biotinylé ; et
(iv) un réactif pour la quantification de la quantité de biotine.

7. Kit selon la revendication 6, dans lequel la matrice d'ARN est constituée de 100 à 500 bases, l'oligo désoxythyminenucléotide est constitué de 5 à 25 bases, et la matrice d'ARN et l'oligodésoxythyminenucléotide sont contenus comme un produit d'hybridation formé par l'hybridation de la matrice d'ARN de l'oligodésoxythyminenucléotide en un rapport molaire de 1 à 1,5-2 000 000.

8. Kit selon la revendication 6, dans lequel la matrice d'ARN est constituée de 100 à 500 bases, l'oligodésoxythyminenucléotide est constitué de 5 à 25 bases, et la matrice d'ARN et l'oligodésoxythyminenucléotide sont contenus comme un produit d'hybridation formé par l'hybridation de la matrice d'ARN et de l'oligodésoxythyminenucléotide en un rapport molaire de 1 à 15-20 000.

9. Kit selon la revendication 6, dans lequel la matrice d'ARN est constituée de 100 à 500 bases, l'oligodésoxythyminenucléotide est constitué de 5 à 25 bases, et la matrice d'ARN et l'oligodésoxythyminenucléotide sont contenus comme un produit d'hybridation formé par hybridation de la matrice d'ARN et de l'oligodésoxythyminenucléotide en un rapport molaire de 1 à 150-2000.

10. Kit selon la revendication 6 comprenant en plus :
(v) du désoxythymidine triphosphate.
